# EUROPEAN PATENT SPECIFICATION

(11) **EP 2 418 492 B1**
(45) Date of publication and mention of the grant of the patent: **10.10.2018**
(21) Application number: 10172415.1
(22) Date of filing: 10.08.2010
(51) Int. Cl.: G01N 33/94, C07K 16/44

(54) **Immunoreagent for meprobamate**
Immunoreagens für Meprobamate
Immunoréactif pour le méprobamate

(43) Date of publication of application: 15.02.2012
(73) Proprietor: Randox Laboratories Ltd., Crumlin, County Antrim BT29 4QY (GB)
(72) Inventor: McConnell, Ivan, Crumlin, Antrim BT29 4QY (GB); Benchikh, Elouard, Crumlin, Antrim BT29 4QY (GB); Fitzgerald, Peter, Crumlin, Antrim BT29 4QY (GB); Lowry, Philip, Crumlin, Antrim BT29 4QY (GB)

(56) References cited:
- GB-A- 1 401 297
- C. MOORE ET AL: "Analysis of pain managemnet drugs, specifically fentanyl, in hair: Application to forensic specimens.", FORENSIC SCIENCE INTERNATIONAL, vol. 176, no. 1, 2008, pages 47-50, XP002617009, Ireland ISSN: 0379-0738
- ALLEN L V JR ET AL: "SPECIFICITY OF THE ENZYME MULTIPLIED IMMUNOASSAY TECHNIQUE DRUG ABUSE URINE ASSAY METHODS", CLINICAL TOXICOLOGY, US, vol. 18, no. 9, 1 January 1981 (1981-01-01), pages 1043-1066, XP008131570, ISSN: 0009-9309

## Description

### FIELD OF THE INVENTION

The invention relates to analytical methods for drug detection, and provides antibodies for the detection and / or determination of meprobamate and carisoprodol.

### BACKGROUND TO THE INVENTION

Carisoprodol and meprobamate are centrally-acting drugs prescribed as muscle-relaxants and sedatives. Both have been implicated with abuse, drug overdose and driving impairment especially meprobamate which possesses a Schedule IV rating under the Controlled Substances Act in the US. Meprobamate is also the principal metabolite of carisoprodol and is rapidly formed upon carisoprodol ingestion. Carisoprodol has a serum half-life of under 2 hours with less than 1% of a single orally administered carisoprodol tablet (350 mg) excreted unchanged in urine (Olsen et al; Baselt R.C. 2008). Meprobamate has a plasma half-life of 6-16 hours and approximately 10-12% is excreted unchanged in the urine (Meprobamate: AHFS Drug Information). GB 1401297 describes a novel enzyme-amplification detection system for competitive immunoassays and lists a multitude of hypothetical targets suitable for detection by the system including centrally-acting drugs such as tranquilizers; this enzyme multiplied immunoassay technique (EMIT) is configured to detect multiple targets including drugs of abuse and the specificity of the system has been tested against various potential drug cross-reactants including meprobamate and carisoprodol (Allen 1981). There are three commercially available immunoassay test kits for carisoprodol. However, due to the rapid metabolism of carisoprodol these kits are unsuitable for long-term detection of the drug in individuals as it is not detectable in serum beyond 12 hours (Olsen et al 1994; Moore et al 2008). There are no known immunoassays targeting meprobamate. Tests for meprobamate utilise gas chromatography linked to mass spectrometry (GC-MS) and gas chromatography (GC) which are expensive, require specialist staff and necessitate sample pre-treatment steps (Daval et al 2006; Gaillard et al 1997). There is a need for a cheap, practical and sensitive meprobamate-specific assay enabling an extended detection window for carisoprodol and meprobamate ingestion. The current invention describes such an assay.

### Bibliography

Allen L.V. (1981). Clin. Toxicol., 18: 1043-165.
Baselt R.C. (2008). In Disposition of Toxic Drugs and Chemicals in Man. 8th edition,
Biomedical Publications, Foster City, California, 2008, pp.920-2.
Daval et al. (2006). J. Anal. Toxicol. 30: 302-5.
Gaillard Y et al. (1997). Forensic Sci Int., 86: 173-80.
Moore C. et al. (2008). Forensic Sci Int., 176: 47-50.
Olsen H. et al. (1994). Ther. Drug Monit., 16: 337-340.
Robertson M.D. and Marinetti L.J. (2003). Forensic Sci. Rev., 15: 1-10.

### DETAILED DESCRIPTION OF THE INVENTION

The present disclosure provides immunogens comprising meprobamate coupled by way of a crosslinker through one of the nitrogen atoms to an antigenicity-conferring carrier material (accm). The immunogens correspond to structures of the formula of Structure I

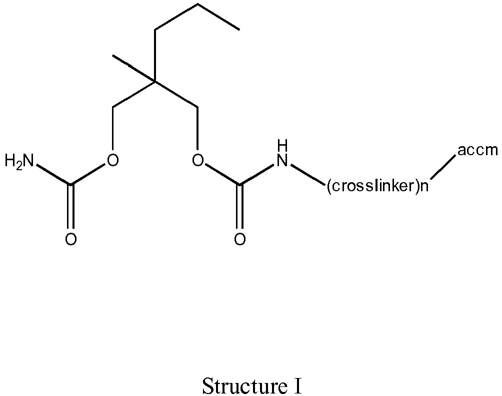

The immunogens are formed from the corresponding haptens (examples of which are to be found in the Methods section). Addition of a crosslinker to a nitrogen atom of meprobamate produces the (*R*,*S*)-hapten due to formation of an asymmetric centre. For the purpose of the invention the racemic hapten is used to prepare the described immunogens. Alternatively, either of the individual haptenic stereoisomers (i.e. 'R' or 'S') could be used for immunogen formation. The immunogens described in the invention are synthesised from the corresponding hapten by preferably attaching a crosslinker to an accm. A crosslinker, well known in the art, prior to attachment to meprobamate is normally a bifunctionalised molecule that bridges two discrete molecules (in this case meprobamate and the accm). Optionally, the immunogen lacks a crosslinking group and meprobamate is bound directly to a suitable accm.

The crosslinker is -Y-Z- , where Y is a C₁-C₁₀, preferably a C₁-C₆, substituted or unsubstituted straight chain alkylene moiety, or arylene moiety; Z (before conjugation with the accm) is selected from a carboxy, a dithiopyridyl, a maleimide, an amino, a hydroxyl, a thiol, a thioester or an aldehyde moiety, most preferably a carboxy moiety. Where Z, before conjugation to the accm is a carboxylic acid (COOH), the oxygen of the hydroxyl group combines first with DCC and then NHS to form an ester with a powerful leaving group. Nucleophilic attack on the carbonyl group (C=O) of the ester functionality by a free amine group on the accm results in an amide bond and formation of the desired immunogen. Conjugate formation follows a similar mechanism using EDC and sulfo-NHS. The skilled reader is referred to Bioconjugate Techniques, G. Hermanson ed., Academic Press, 1996, 785 pp for details of the interaction between the Z reactive group and the accm. Most advantageously, Z, before conjugation with an accm to produce an immunogen, is a carboxy (COOH) moiety.

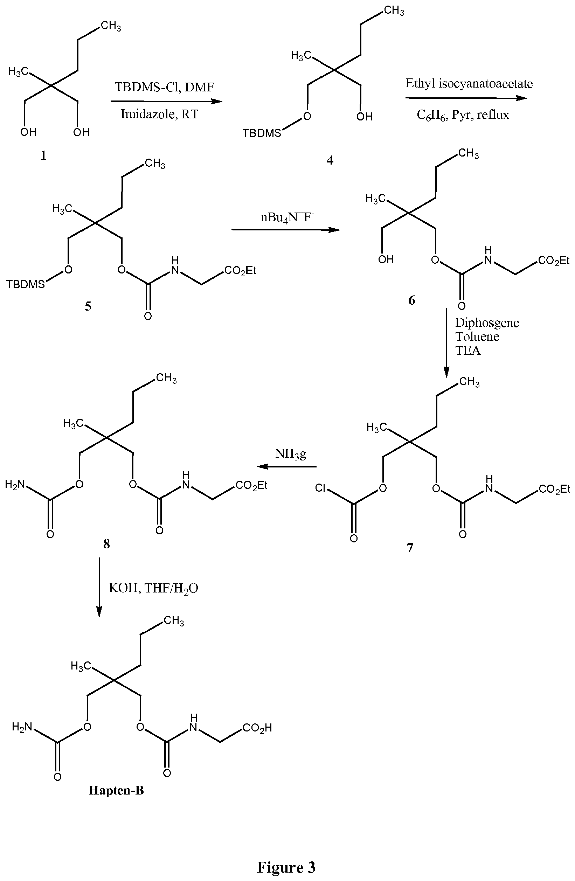

The immunogens are prepared by coupling to a modified or non-modified accm. Preferably the accm contains poly(amino acid) segments and includes proteins, protein fragments, glycoproteins, synthetic polypeptides or semi-synthetic polypeptides. Illustrative examples of useful antigenicity-conferring carrier materials are bovine serum albumin (BSA), egg ovalbumin, bovine gamma globulin, bovine thyroglobulin (BTG), keyhole limpet haemocyanin (KLH) etc. Alternatively, synthetic poly(amino acids) having a sufficient number of available amino groups, such as lysine, may be employed, as may other synthetic or natural polymeric materials bearing reactive functional groups. The immunogens obtained are then administered to mammalian hosts to elicit production of specific antibodies, optionally polyclonal antibodies, which are then used to develop immunoassays for meprobamate and carisoprodol, employing labelled conjugates as detection reagents. Preferably, the immunogen is N-carboxymethylmeprobamate (hapten-B Figure 3) coupled to an accm, the accm optionally selected from bovine serum albumin (BSA) and bovine thyroglobulin (BTG).

The present invention concerns antibodies raised against the immunogens of the present invention, the antibodies being capable of binding with at least one structural epitope of meprobamate, carisoprodol and related molecules.

In a further aspect, the present invention concerns antibodies having specificity for a structural epitope of meprobamate and having cross-reactivity for carisoprodol. Antibodies that are specific for a certain molecule implies that the antibodies bind preferably to that molecule compared to other molecules. This is commonly represented by the skilled person using the cross-reactivity concept, in which the antibody-specific molecule has 100 % cross-reactivity to the antibody and all other molecules have a cross-reactivity of less than 100% to the antibody. The antibodies of the current invention are specific to an epitope of meprobamate and may have cross-reactivity to an epitope of carisoprodol. The cross-reactivity of the antibodies to carisoprodol is >10%, preferably >30%, most preferably >50% but is always less than the cross-reactivity to meprobamate. Similarly, the meprobamate-specific antibodies of the invention can cross-react (less than 100% compared to meprobamate) with mebutamate, tybamate and similar N- monoalkylated analogues of meprobamate.

The disclosure shows also a method for detecting or determining meprobamate and/or carisoprodol in an *in vitro* sample, the method comprising contacting the sample with at least one conjugate, and with at least one antibody of the present invention; detecting or determining bound conjugate; and deducing from a calibration curve the presence of, or the amount of meprobamate and/or carisoprodol in the sample. Detecting implies the qualitative analysis of the presence or absence; determining means the quantitative analysis. As is known by the skilled man, for a quantitative competitive immunoassay incorporating an antibody which is able to bind to an epitope (whether the same or different) of more than one molecule, the determined value is derived from calibrator or standard equivalents Within the context of the current invention calibrator/standard are synonymous unless otherwise stated. In order to derive quantitative values for the individual molecules meprobamate and carisoprodol, a two antibody immunoassay approach could be used, incorporating, for example, the antibodies of the invention and an additional antibody that binds either meprobamate or carisoprodol, but not both. Table 1 shows the cross-reactivity profile of an antibody specific to carisprodol produced by the inventors which when utilised with the antibody of the invention would enable individual quantification of both meprobamate and carisoprodol.

Also disclosed is a kit for detecting meprobamate and/or carisoprodol, the kit including at least one antibody of the present invention and optionally including at least one conjugate. Alternatively, the kit is for determining meprobamate and/or carisoprodol, the kit including at least one antibody of the present invention and optionally including at least one conjugate. The kit may optionally include instructions for the use of said conjugates and said antibodies for determining the amount of each of meprobamate and/or carisoprodol in a sample. The sample can be any biological fluid but is preferably whole blood.

### Methods and Results

### General Procedure for MALDI-TOF Analysis of Immunogens.

In order to confirm that adequate conjugation to an antigenicity-conferring carrier material has been achieved, prior to immunisation, each immunogen is evaluated using matrix-assisted UV laser desorption /ionisation time-of-flight mass spectroscopy (MALDI-TOF MS). MALDI-TOF mass spectrometry was performed using a Voyager STR Biospectrometry Research Station laser-desorption mass spectrometer coupled with delayed extraction. An aliquot of each sample to be analysed was diluted in 0.1% aqueous trifluoroacetic acid (TFA) to create 1mg/ml sample solutions. Aliquots (1µl) were analysed using a matrix of sinapinic acid and bovine serum albumin (Fluka) was used as an external calibrant.

### Preparation of Antisera

In order to generate polyclonal antisera, the immunogen of the present invention is mixed with Freund's Adjuvant and the mixture is injected into a host animal, such as rabbit, sheep, mouse, guinea pig or horse. Further injections (boosts) are made and serum is sampled for evaluation of the antibody titre. When the optimal titre has been attained, the host animal is bled to yield a suitable volume of specific antiserum. The degree of antibody purification required depends on the intended application. For many purposes, there is no requirement for purification, however, in other cases, such as where the antibody is to be immobilised on a solid support, purification steps can be taken to remove undesired material and eliminate non-specific binding. The specific antibodies prepared in this invention are useful as reagents in immunoassays for the detection or determination of meprobamate and carisoprodol in biological fluids. The antibodies of the present invention are also capable of binding with N-monoalkylated analogues of meprobamate.

### Example 1: Preparation of 2-methyl-2-hydroxymethylpentyl N-isopropylcarbamate 2

To a stirred solution of 2-methyl-2-propyl-1,3-propanediol 1 (19.83g, 0.15mol) in anhydrous toluene (250ml) was added isopropyl isocyanate (16.21ml, 0.165mol). The mixture was heated at reflux for 5 h. The reaction mixture was allowed to cool to room temperature, water (100ml) was added to the mixture and the two layers were separated. The aqueous layer was extracted with ethyl acetate (1 x 100ml) and the combined organic layers were washed with water (100ml), brine (100ml), dried over sodium sulphate, filtered and concentrated *in vacuo.* The crude product (26.6g) was purified by chromatography on silica gel (hexane/ethyl acetate: 1/1) to give 2-methyl-2-hydroxymethylpentyl N-isopropylcarbamate **2** (22.5g, 69%) as a yellow oil.

### Example 2: Preparation of 2-methyl-2-propyl-1,3-propanediol N-isopropyl-N-ethoxycarbonylmethyldicarbamate 3

To a stirred solution of **2** (21.731g, 0.1mol) in anhydrous toluene (300ml) was added pyridine (10ml) and ethyl isocyanatoacetate (13.5ml, 0.12mol) and the mixture was heated at reflux overnight. The solution was then cooled at room temperature, water was added (150ml) and the two layers separated. The aqueous layer was extracted by ethyl acetate (2 x 100ml) and the combined organic layers were washed with water (2 x 100ml), brine (100ml), dried over sodium sulphate, filtered and concentrated to dryness to give the pure compound **3** as a yellow oil (20.7g, 59.7%).

### Example 3: Preparation of 2-methyl-2-propyl-1,3-propanediol N-isopropyl-N-carboxymethyldicarbamate (N-{carboxymethyl)carisoprodol/Hapten-A)

Compound **3** (3.46g, 0.01mol) was dissolved in a mixture of tetrahydrofuran (THF) (100ml) and water (100ml). Potassium hydroxide (KOH) (4.37g, 0.032mol) was added and the mixture stirred overnight at room temperature. The THF was removed under reduced pressure and the aqueous solution acidified to pH 2 by HCl (2N). The white solid formed was filtered, washed with water and dried under vacuum overnight to give Hapten-A (2.9g, 91%).
NMR ¹³C (δ: ppm): 172.47, 158.20, 157.19, 68.79, 42.92, 41.96, 37.66, 36.85, 21.80, 18.17, 16.33 and 14.13

### Example 4: Conjugation of Hapten-A to BSA (Immunogen 1)

To a solution Hapten-A (35.82mg, 0.1128mmol) in DMF (1.0ml) was added N,N-dicyclohexylcarbodiimide (DCC) (25.35mg, 0.123mmol) and N-hydroxysuccinimide (14.13mg, 0.123mmol) and the mixture was stirred at room temperature overnight. The dicyclohexylurea formed was removed by filtration and the solution was added dropwise to a solution of BSA (150mg, 2.3µmol) in 50mM sodium bicarbonate solution (10ml, pH 8.5). The mixture was stirred overnight at 4°C. The solution was dialysed against 50mM phosphate buffer pH 7.2 (3 changes) for 24 h at 4°C, and freeze-dried to give 141mg of Immunogen 1. MALDI results showed 31.23 molecules of hapten -A had been conjugated to one molecule of BSA.

### Example 5: Conjugation of Hapten-A to BTG (Immunogen 2)

To a solution of Hapten-A (43.91mg, 0.135mmol) in DMF (1.0ml) was added N, N-dicyclohexylcarbodiimide (DCC) (30.7mg, 0.149mmol) and N-hydroxysuccinimide (17.13mg, 0.149mmol) and the mixture stirred at room temperature overnight. The dicyclohexylurea formed was removed by filtration and the solution was added dropwise to a solution of BTG (150mg, 2.25υmol) in 50mM sodium bicarbonate solution (10ml, pH 8.5). The mixture was then stirred overnight at 4°C. The solution was then dialysed against 50mM phosphate buffer pH 7.2 (3 changes) for 24 h at 4°C, and freeze-dried to give 145mg of Immunogen 2.

### Example 6: Conjugation of Hapten-A to HRP

EDC hydrochloride (10mg) was dissolved in water (0.5ml) and immediately added to a solution of Hapten-A (2mg) in DMF (0.2ml). After mixing, this solution was added dropwise to a solution of HRP (20mg) in water (1ml). Sulfo-NHS (5mg) was added and the reaction mixture was incubated in the dark at room temperature overnight. Excess hapten was removed with double PD-10 columns (Pharmacia) in series, pre-equilibrated with PBS at pH 7.2. The hapten-HRP conjugate was then dialysed overnight against 10L of PBS at pH 7.2 at 4°C.

### Example 7: Preparation O-terbutyldimethylsilyl 2-methyl-2-propyl-1,3-propanediol 4

To a stirred solution of 2-methyl-2-propyl-1,3-propanediol **1** (19.83g, 0.15mol) in DMF (200ml) at 0°C was added imidazole (20.42g, 0.3mol) and TBDMS-CL (24.9g, 0.165mol) and the mixture was stirred at room temperature overnight. Water (300ml) was added to the mixture and the solution was extracted with diethyl ether (2 x 300ml). The combined diethyl ether layers were washed by water (1 x 200ml), brine (1 x 100ml), dried over sodium sulphate, filtered and concentrated to dryness. The crude product obtained was purified by flash chromatography on silica gel using 15% ethyl acetate/ 85% hexane to give O-terbutyldimethylsilyl 2-methyl-2-propyl-1, 3-propanediol **4** (22.1g, 60%) as a colourless oil.

### Example 8: Preparation of 2-(O-terbutyldimethylsiloxymethyl)-2-methylpentyl N-ethoxycarbonylmethylcarbamate 5

To a stirred solution of **4** (11.0g, 44.71mmol) in anhydrous toluene (200ml) was added pyridine (10ml) and ethyl isocyanatoacetate (6.6ml, 58.12mmol) and the mixture heated at reflux overnight. The solution was cooled at room temperature, water added (150ml) and the two layers separated. The aqueous layer was extracted with ethyl acetate (2 x 100ml) and the combined organic layers were washed with water (2 x 100ml), brine (100ml), dried over sodium sulphate, filtered and concentrated to dryness to give 2-(O-terbutyldimethylsiloxymethyl)-2-methylpentyl N-ethoxycarbonylmethylcarbamate **5** (17.27g) as a milky liquid.

### Example 9: Preparation of 2-hydroxymethyl-2-methylpentyl N-ethoxycarbonylmethylcarbamate 6

To s stirred solution of **5** (17.0g, 45.33mmol) in THF (300ml) at 0°C was added tetrabutylammonium fluoride (17.16g, 54.4mmol) and the mixture stirred at room temperature for 4 h. The solvent was removed *in vacuo* and the residue purified by flash chromatography on silica gel using 30% ethyl acetate / 70% hexane to give the pure compound **6** as a clear viscous oil (9.2g, 77.7%).

### Example 10: Preparation on 2-methyl-2-propyl-1,3-propanediol N-ethoxycarbonylmethyldicarbamate 8

To a stirred solution of compound **6** (9.0g, 34.5mmol) in anhydrous toluene (150ml) and TEA (5.2ml) at 0°C was added diphosgene (2.1ml, 17.25mmol). The mixture was stirred at room temperature for 1 h. Dichloromethane (150ml) was added and the mixture cooled to -78°C and ammonia gas bubbled through for 10 min. The mixture was allowed to warm to room temperature and the solvent removed *in vacuo.* The residue obtained was triturated with ethyl acetate and the mixture filtered. The filtrate was concentrated to dryness and triturated with hexane/diethyl ether (50/50) to give 2-methyl-2-propyl-1,3-propanediol N-ethoxycarbonylmethyldicarbamate **8** as a white solid (8.3g, 79%).

### Example 11: Preparation of 2-methyl-2-propyl-1,3-propanediol N-carboxymethyldicarbamate (N-(carboxymethyl)meprobamate/Hapten-B)

Compound **8** (4.8g, 15.8mmol) was dissolved in a mixture of tetrahydrofuran (THF) (100ml) and water (100ml). Potassium hydroxide (KOH) (5.3g, 47.5mmol) was added and the mixture stirred overnight at room temperature. The THF was removed under reduced pressure, the aqueous solution acidified to pH 3-4 with HCl (2N) and the solution extracted with ethyl acetate (2 x 10ml). The combined organic extracts where washed with water (1 x 100ml), brine (1 x 100ml), dried over sodium sulphate, filtered and concentrated to dryness to give Hapten-B as a clear gum (3.5g, 80%). NMR ¹³C (δ ppm): 174.65, 160.93, 159.92, 70.26, 69.90, 43.74, 39.13, 38.29, 19.72, 17.84 and 15.85.
MS: [M-H]⁺: 275.1241.

### Example 12: Conjugation of Hapten-B to BSA (Immunogen 3)

To a solution Hapten-B (31.05mg, 0.1125mmol) in DMF (1.0ml) was added N,N-dicyclohexylcarbodiimide (DCC) (25.35mg, 0.123mmol) and N-hydroxysuccinimide (14.13mg, 0.123mmol) and the mixture stirred at room temperature overnight. The dicyclohexylurea formed was removed by filtration and the solution was added drop-wise to a solution of BSA (150mg, 2.3µmol) in 50mM sodium bicarbonate solution (pH 8.5) (10ml). The mixture was stirred overnight at 4°C. The solution was dialysed against 50mM phosphate buffer pH 7.2 (3 changes) for 24 h at 4°C, and freeze-dried to give 135mg of Immunogen 3. MALDI results showed 27.04 molecules of Hapten-B had been conjugated to one molecule of BSA.

### Example 13: Conjugation of Hapten-B to BTG (Immunogen 4)

To a solution of Hapten-B (37.29mg, 0.135mmol) in DMF (1.0ml) was added N, N-dicyclohexylcarbodiimide (DCC) (30.7mg, 0.149mmol) and N-hydroxysuccinimide (17.13mg, 0.149mmol) and the mixture stirred at room temperature overnight. The dicyclohexylurea formed was removed by filtration and the solution added dropwise to a solution of BTG (150mg, 2.25υmol) in 50mM sodium bicarbonate solution (10ml, pH 8.5). The mixture was stirred overnight at 4°C then dialysed against 50mM phosphate buffer pH 7.2 (3 changes) for 24 h at 4°C, and freeze-dried to give 140mg of Immunogen 4.

### Example 14: Conjugation of Hapten-B to HRP

EDC hydrochloride (10mg) was dissolved in water (0.5ml) and added to a solution of Hapten-B (2mg) in DMF (0.2ml). After mixing, the solution was added dropwise to a solution of HRP (20mg) in water (1ml). Sulfo-NHS (5mg) was added and the reaction mixture was incubated in the dark at room temperature overnight. Excess hapten was removed with double PD-10 columns (Pharmacia) in series, pre-equilibrated with PBS at pH 7.2. The hapten-HRP conjugate was then dialysed overnight against 10L of PBS at pH 7.2 at 4°C.

### Example 15: Preparation of antibodies to Immunogen 4

An aqueous solution of Immunogen **4** was formulated with Freund's Complete Adjuvant (FCA) to form an emulsion consisting of 2 mg/ml immunogen in 50% (v/v) FCA. Three sheep were immunised with this emulsion (1° immunisation), 0.25 ml being intramuscularly injected at each of four sites in the rump of each animal. Subsequent immunizations (boosts) contained 1mg/ml immunogen. All boosts were emulsified in 50% (v/v) Freund's Incomplete Adjuvant (FIA) and were administered in the same manner as the 1° immunisation, at monthly intervals for 1 year. Blood sampling took place 7 to 14 days after each boost. Each sample was processed to produce antiserum, which was further purified by caprylic acid and ammonium sulfate precipitation to yield an immunoglobulin (Ig) fraction. The Ig fraction was evaluated by competitive ELISA microtiter plate assay, as described in Example 16.

### Example 16: Development of ELISAs for Meprobamate and Carisprodol

The wells of an enhanced binding 96 well polystyrene microtiter plate were coated with a mixture of the Ig fractions of the antisera raised to Immunogen **4,** diluted in 10 mM Tris, pH8.5 (125 µl/well). The appropriate antibody coating dilution was determined using standard ELISA checkerboard techniques. The plate was incubated for 2 hours at 37°C, washed 4 times with Tris buffered saline containing Tween 20 (TBST) and tapped dry. Standard solutions of meprobamate, carisoprodol and mebutamate were prepared in TBST at 0, 0.1, 1.0, 5, 10, 25, 50 and 100 ng/ml, and 50 µl of each was added to the appropriate wells.

The conjugate prepared in Example 14 diluted in Tris buffer (pH 7.2) containing EDTA, D-mannitol, sucrose, thimerosal and BSA, was added to each of the wells. The appropriate dilution of conjugate was also determined using standard ELISA checkerboard techniques. The plate was incubated at 25°C for 1 hour. Excess unbound conjugate was removed by washing 6 times over a 10 minute period with TBST. 125 µl of tetramethylbenzidine (TMB) substrate solution was added to each well of the plate that was then incubated for 20 minutes in the dark at room temperature. The reaction was terminated by addition of 125 µl 0.2M H₂SO₄ to each well. The absorbance was then measured at 450 nm using a microtiter plate reader.

### Results

The calculated IC₅₀ and CR values for meprobamate and carisoprodol were 8.64 ng/ml and 100% and 15.15 ng/ml and 57%, respectively. The antibody displayed a cross-reactivity of 7% to the minor prescription drug mebutamate. Tybamate, of similar structure to carisoprodol, is expected to display cross-reactivity to the antibody, as would other N-monoalkylated meprobamate derivatives in which the alkyl chain is of short to medium length (approximately C₁-C₁₀). Table 1 compares the IC₅₀ and CR values of commercially available immunoassays and the immunoassay of the current invention. As can be seen, the antibody of the invention enables an immunoassay that is uniquely specific for meprobamate.

**Table 1 Specificity and sensitivity of carisoprodol-specific antibody raised from Immunogen 2**

| **Antibody property** | **Value** |
|---|---|
| IC₅₀ carisoprodol (ng/ml) | 15 |
| IC₅₀ meprobamate (ng/ml) | 8 |
| %CR carisoprodol | 57 |
| %CR meprobamate | 100 |

**Table 2 Specificity and sensitivity comparisons of meprobamate and carisoprodol immunoassays**

| **Antibody property** | **Randox** | **Immunalysis*** | **Neogen**** | **IDS** |
|---|---|---|---|---|
| IC₅₀ carisoprodol (ng/ml) | 15 | 25 | nk | 0.6 |
| IC₅₀ meprobamate (ng/ml) | 8 | 132 | nk | >6000 |
| %CR carisoprodol | 57 | 100 | nk | 100 |
| %CR meprobamate | 100 | 19 | nk | <0.01 |

| | | | | |
|---|---|---|---|---|
| IC₅₀ = standard concentration which produces 50% B/B₀; %CR = percentage cross-reactivity based on 100% specificity to meprobamate (B = UV absorbance at 450 nm at x ng/ml calibrator concentration; B₀ = absorbance at 450 nm at 0 ng/ml calibrator concentration). *Data from Robertson and Marinetti, 2003 ** described as a carisoprodol immunoassay; nk= not known | | | | |

### DESCRIPTION OF FIGURES

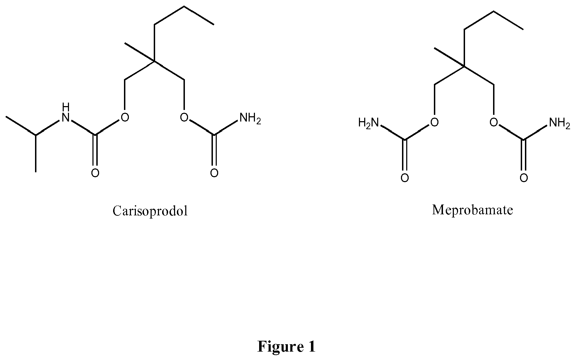

FIGURE 1 Structures of meprobamate and carisoprodol

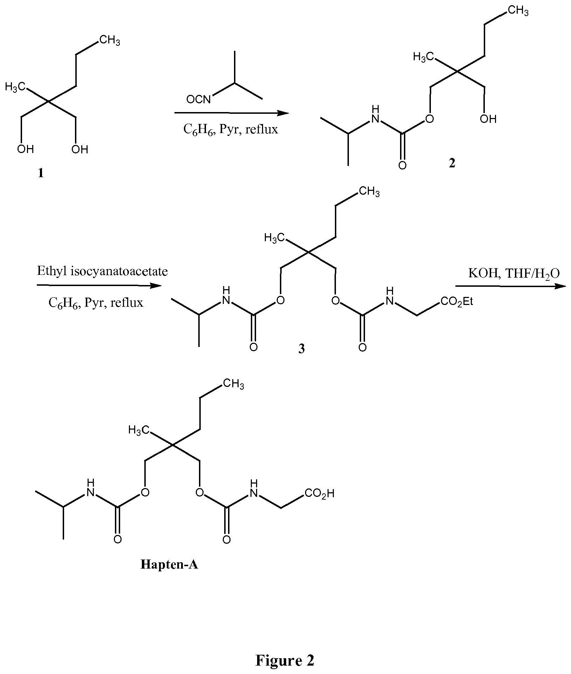

FIGURE 2 Preparation of a carisoprodol hapten (Hapten-A)
FIGURE 3 Preparation of a meprobamate hapten (Hapten-B)

## Claims

1. An antibody raised against an immunogen of the structure 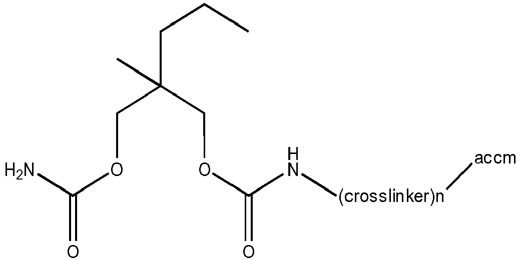 where n=0 or 1, the accm is an antigenicity-conferring carrier material and the crosslinker is -Y-Z- where Y is a C₁-C₆ unsubstituted straight chain alkylene moiety; Z (before conjugation to the accm) is selected from a carboxy, a dithiopyridyl, a maleimide, an amino, a hydroxyl, a thiol, a thioester or an aldehyde moiety, and links a nitrogen atom of meprobamate to the accm in which the accm is chosen from bovine thyroglobulin, bovine serum albumin, keyhole limpet haemocyanin, egg ovalbumin and bovine gamma globulin, the antibody being specific for a structural epitope of meprobamate **characterised by** having cross-reactivity to carisoprodol.

2. The antibody of Claim 1 in which the immunogen has Y = methylene and Z (before conjugation to the accm) = a carboxy group.

3. The antibody of the preceding claims which has a cross-reactivity to carisoprodol that is greater than 10%, preferably greater than 30%, most preferably greater than 50%, but is less than 100%.

## Patentansprüche

1. Ein Antikörper gegen ein Immunogen der Struktur 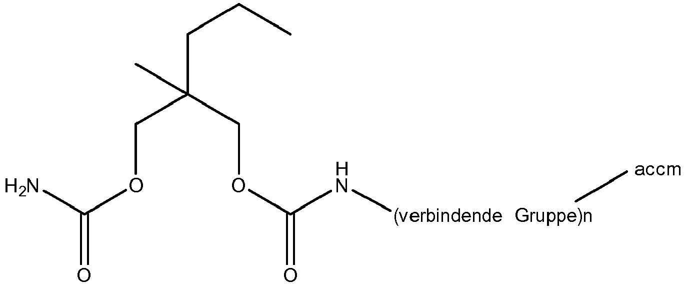 wobei n = 0 oder 1 ist, das accm ein Antigen-verleihendes Trägermaterial ist und der verbindende Gruppe ist -Y-Z-, wobei Y eine unsubstituierte geradkettige C₁-C₆ Alkyleneinheit ist; Z (vor der Konjugation an die accm) ist ausgewählt aus einer Carboxyl-, einer Dithiopyridyl-, einer Maleimid-, einer Amino-, einer Hydroxyl-, einer Thiol-, einer Thioester- oder einer Aldehydeinheit und verbindet ein Stickstoffatom von Meprobamat mit dem accm, in dem die accm ausgewählt ist aus Rinderthyroglobulin, Rinderserumalbumin, Schlüssellochnapfschnecken-Hämocyanin, Ei-Ovalbumin und Rinder-Gammaglobulin, wobei der Antikörper spezifisch für ein Strukturepitop von Meprobamat ist, das durch Kreuzreaktivität mit Carisoprodol gekennzeichnet ist.

2. Antikörper nach Anspruch 1, in dem das Immunogen Y = Methylen und Z (vor der Konjugation mit dem Accum) = eine Carboxygruppe aufweist.

3. Antikörper nach den vorhergehenden Ansprüchen, der eine Kreuzreaktivität zu Carisoprodol aufweist, die größer als 10%, vorzugsweise größer als 30%, am meisten bevorzugt größer als 50% ist, aber weniger als 100% beträgt.

## Revendications

1. Un anticorps généré contre un immunogène de la structure 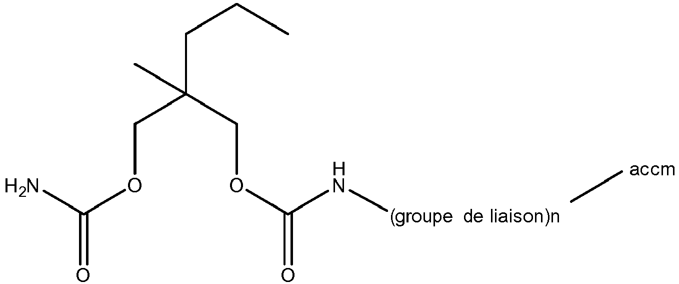 où n = 0 ou 1, l'accm est une substance support conférant une antigénicité et le groupe de liaison est -Y-Z- où Y est un groupement alkylène à chaîne droite non substitué en C₁-C₆; Z (avant la conjugaison à l'accm) est choisi parmi un carboxy, un dithiopyridyle, un maléimide, un amino, un hydroxyle, un thiol, un thioester ou un fragment aldéhyde, et lie un atome d'azote du méprobamate au composé dans lequel le est choisi parmi la thyroglobuline bovine, la sérumalbumine bovine, l'hémocyanine de patelle, l'ovalbumine d'oeuf et la gamma globuline bovine, l'anticorps étant spécifique d'un épitope structural du méprobamate **caractérisé par** une réactivité croisée avec le carisoprodol.

2. Anticorps selon la revendication 1, dans lequel l'immunogène a Y = méthylène et Z (avant la conjugaison à l'accm) = un groupe carboxy.

3. Anticorps selon les revendications précédentes qui a une réactivité croisée avec le carisoprodol qui est supérieure à 10%, de préférence supérieure à 30%, de manière préférée entre toutes supérieure à 50%, mais inférieure à 100%.
